# EUROPÄISCHE PATENTANMELDUNG

(11) **EP 4 650 340 A1**
(43) Veröffentlichungstag der Anmeldung: **19.11.2025**
(21) Anmeldenummer: 24176556.9
(22) Anmeldetag: 17.05.2024
(51) Int. Cl.: C07C 49/90, C07C 45/54, C07C 51/567, C07C 51/573, C07C 57/04, C07C 53/12, C07C 51/56

(54) **VERFAHREN ZUR HERSTELLUNG VON UNGESÄTTIGTEN SYMMETRISCHEN CARBONSÄUREANHYDRIDEN**

(71) Anmelder: Evonik Operations GmbH, 45128 Essen (DE)
(72) Erfinder: KRILL, Steffen, 64367 Mühltal Niederbeerbach (DE); KAY, Alexander, 64342 Seeheim-Jugenheim (DE); TRESKOW, Marcel, 64293 Darmstadt (DE)
(74) Vertreter: Evonik Patent Association

(57) **Zusammenfassung**

Die Erfindung betrifft ein Verfahren zur gleichzeitigen Herstellung von symmetrischen Carbonsäureanhydriden der allgemeinen Formeln R-C(O)-O-C(O)-R (I) und R'R"HC-C(O)-O-C(O)-CHR'R" (II) durch Reaktion einer ungesättigten Carbonsäure mit einem Keten wobei (a) die ungesättigte Carbonsäure (III) in einem ersten Reaktionsbereich mit dem Keten zu einer Roh-Anhydridmischung umgesetzt wird, (b) die in Schritt (a) erhaltene Roh-Anhydridmischung in einem zweiten Reaktionsbereich vorzugsweise in Gegenwart eines Katalysators weiter umgesetzt wird, (c) die symmetrischen Anhydride (I) und (II) im Wege einer Rektifikation aus der in Schritt (b) erhaltenen Mischung isoliert werden, und (d) bei der Rektifikation in Schritt (c) anfallendes, nicht umgesetztes Mischanhydrid in den ersten Reaktionsbereich und/oder in den zweiten Reaktionsbereich zurückgeführt wird.

## Beschreibung

### Gebiet der Erfindung

Die Erfindung beschreibt ein Verfahren zur gleichzeitigen/simultanen Herstellung von ungesättigten symmetrischen Carbonsäureanhydriden der allgemeinen Formeln R-C(O)-O-C(O)-R (I) und R'R"HC-C(O)-O-C(O)-CHR'R" (II) durch Reaktion einer ungesättigten Carbonsäure mit einem Keten.

### Stand der Technik

In DE-A-3510035 wird ein Verfahren zur kontinuierlichen Herstellung von ungesättigten Carbonsäureanhydriden durch säurekatalysierte Umanhydridisierungsreaktion von Essigsäureanhydrid mit einer ungesättigten Carbonsäure im Mittelteil einer Destillationskolonne beschrieben. Zur Erreichung eines vollständigen Umsatzes wird Essigsäureanhydrid in einem Überschuss von 0.1 bis 0.5 Mol pro Mol Carbonsäure eingesetzt, wobei dann am Kolonnenkopf eine Mischung aus Essigsäure und Essigsäureanhydrid anfällt, also keine reine Essigsäure gewonnen wird. Das auf diese Weise gebildete Produkt ist jedoch durch den Katalysator verunreinigt, der erst in einem weiteren Verfahrensschritt entfernt werden muss.

In US-A-4,857,239 wird ein Verfahren zur Herstellung von Methacrylsäureanhydrid beschrieben, wobei das Molverhältnis von Methacrylsäure zu Essigsäureanhydrid 2.1 bis 3 beträgt und ein Polymerisationsinhibitor in die Destillationskolonne zugegeben wird. Das Verfahren wird diskontinuierlich betrieben. Nachteilig ist darüber hinaus, dass das im Überschuss eingesetzte Edukt ungenutzt anfällt.

In US-A-2003/001827 wird ein diskontinuierliches Verfahren zur Herstellung von Methacrylsäureanhydrid beschrieben, wobei das anfängliche Molverhältnis von Methacrylsäure zu Essigsäureanhydrid bevorzugt 9 bis 11 beträgt. Die entstehende Essigsäure wird sofort abgeführt und der freiwerdende Reaktorinhalt mit Essigsäureanhydrid aufgefüllt. Zur Vermeidung einer Polymerisation werden in den Reaktor und in die Kolonne Inhibitoren zugegeben. Es werden viele Nebenprodukte gebildet, die sich nicht vollständig entfernen lassen.

In EP 2 032 519 wird ein Verfahren zur kontinuierlichen Herstellung von ungesättigten Carbonsäureanhydriden beschrieben, bei dem ein vollständiger Umsatz der eingesetzten ungesättigten Carbonsäure erzielt wird und gleichzeitig das gebildete ungesättigte Carbonsäureanhydrid in hoher Reinheit gewonnen wird. Darüber hinaus wird die Polymerisation in allen Bereichen weitgehend vermieden und die Raum-Zeit-Ausbeute der Reaktion im Vergleich zu den vorstehend beschriebenen Verfahren erhöht.

Aufgabe ist es nun ein weiter verbessertes Verfahren zur kontinuierlichen (oder semi-kontinuierlichen) Herstellung von ungesättigten symmetrischen Carbonsäureanhydriden bereitzustellen, das gegenüber EP2032519 eine höhere Raum/Zeit - Ausbeute bezüglich des Anhydrids erreicht. Ferner soll die Bildung von Nebenprodukten (v.a. Essigsäure) unterdrückt werden.

### Zusammenfassung der Erfindung

Den Erfindern ist es überraschenderweise gelungen, ein nebenproduktfreies Verfahren zur gleichzeitigen Herstellung von symmetrischen Carbonsäureanhydriden der allgemeinen Formeln R-C(O)-O-C(O)-R (I) und R'R"HC-C(O)-O-C(O)-CHR'R" (II) entwickeln.

Im Einzelnen betrifft die Erfindung ein Verfahren zur gleichzeitigen (simultanen) Herstellung von symmetrischen Carbonsäureanhydriden der allgemeinen Formel

R-C(O)-O-C(O)-R (I)

und

R'R"HC-C(O)-O-C(O)-CHR'R" (II)

in der R einen ungesättigten organischen Rest mit 2 bis 12 C-Atomen darstellt, worin R' und R" gleich oder verschieden sind und Wasserstoff oder einen C₁ bis C₄-Alkylrest darstellen, und worin R' und R" verschieden von R sind,
durch Reaktion einer ungesättigten Carbonsäure der allgemeinen Formel

   R-COOH (III),
in der R die oben angegebene Bedeutung hat,
mit einem Keten der allgemeinen Formel

   R'R"C=C=O (IV),
in der R' und R" die oben angegebenen Bedeutungen haben, wobei
   (a) die ungesättigte Carbonsäure (III) in einem ersten Reaktionsbereich mit dem Keten (IV) zu einer Roh-Anhydridmischung umgesetzt wird,
   (b) die in Schritt (a) erhaltene Roh-Anhydridmischung in einem zweiten Reaktionsbereich vorzugsweise in Gegenwart mindestens eines Katalysators, weiter umgesetzt wird,
   (c) die symmetrischen Anhydride (I) und (II) im Wege einer Rektifikation aus der in Schritt (b) erhaltenen Mischung isoliert werden, und
   (d) bei der Rektifikation in Schritt (c) anfallendes, nicht umgesetztes Mischanhydrid in den ersten Reaktionsbereich und/oder in den zweiten Reaktionsbereich zurückgeführt wird.

Durch die Kombination der vorstehend genannten Verfahrensmerkmale wird erreicht, dass ein vollständiger Umsatz der Edukte und gleichzeitig eine hohe Reinheit der Produkte erzielt wird und die Polymerisation in allen Bereichen weitgehend vermieden wird, da u.a. lange Verweilzeiten des gebildeten ungesättigten Anhydrids im Kolonnensumpf ausgeschlossen werden.

Aufgrund des vollständigen Umsatzes im ersten Reaktionsbereich bleibt die Rektifikationskolonne nahezu frei von freien Carbonsäuren.

### Detaillierte Beschreibung der Erfindung

Für das erfindungsgemäße Verfahren geeignete ungesättigte Carbonsäuren weisen einen ungesättigten organischen Rest mit 2 bis 12, vorzugsweise 2 bis 6, besonders bevorzugt 2 bis 4 Kohlenstoffatomen auf. Geeignete Alkenylgruppen sind insbesondere die Vinyl-, Allyl-, 2-Methyl-2-propen-, 2-Butenyl-, 2-Pentenyl-, 2-Decenyl, 1-Undecenyl und die 9,12-Octadecadienyl-Gruppe. Besonders bevorzugt sind die Vinyl- und die Allylgruppe.

Zu den besonders bevorzugten Carbonsäuren gehören u.a. (Meth)acrylsäuren. Der Begriff "(Meth)acrylsäure" ist in der Fachwelt bekannt, wobei hierunter neben Acrylsäure und Methacrylsäure auch Derivate dieser Säuren zu verstehen sind. Zu diesen Derivaten gehören unter anderem β-Methylacrylsäure (Butensäure, Crotonsäure), α, β-Dimethylacrylsäure, β-Ethylacrylsäure, α-Chloracrylsäure, α-Cyanacrylsäure, 1-(Trifluormethyl)acrylsäure, sowie β,β-Dimethylacrylsäure. Bevorzugt sind Acrylsäure (Propensäure) und Methacrylsäure (2-Methylpropensäure).

Das molare Verhältnis der Reaktanten, das heißt von ungesättigter Carbonsäure der Formel (III) zu Keten der Formel (IV), beträgt üblicherweise 1:4 bis 1:0,5, vorzugsweise 1:1.

Das ungesättigte Carbonsäureanhydrid der Formel (I) ist vorzugsweise (Meth)acrylsäureanhydrid, hergestellt durch Reaktion von einem Keten der Formel CH₂=C=O und (Meth)acrylsäure. Das Carbonsäureanhydrid der Formel (II) ist vorzugsweise Essigsäureanhydrid.

Im erfindungsgemäßen Verfahren kann das (Meth)acrylsäureanhydrid zwischen dem mittleren und dem unteren Bereich der Rektifikationskolonne und das Essigsäureanhydrid am Kolonnenkopf abgezogen werden.

Geeignete Ketene für das erfindungsgemäße Verfahren besitzen die allgemeine Formel (IV) R'R" C=C=O, worin R' und R" gleich oder verschieden sind und Wasserstoff oder einen C₁ bis C₄-Alkylrest darstellen. Vorzugsweise wird als Keten CH₂=C=O verwendet.

Die Herstellung des Ketens erfolgt nach gängigen Verfahren, die aus der allgemeinen Fachliteratur, beispielsweise aus H. Held, A. Rengstl und D. Mayer, Acetic Anhydride and Mixed Fatty Acid Anhydrides in Ullmann's Encyclopedia of Industrial Chemistry, 6. Aufl., Wiley VCH, Weinheim, 2003, S.184-185, bekannt sind.

Beispielsweise wird das als Reaktant verwendete Keten durch thermische Spaltung von einer Carbonsäure der allgemeinen Formel R'R"CH-COOH (V), worin R' und R" die obengenannte Bedeutung haben, in einem Ketenofen in Anwesenheit eines üblichen Katalysators wie beispielsweise Triethylphosphat erhalten. Die thermische Spaltung erfolgt unter allgemein üblichen Temperatur- und Druckverhältnissen.

Bevorzugt ist die thermische Spaltung von Essigsäure, wobei als Keten CH₂=C=O erhalten wird.
Figur 1 zeigt eine Anlage zur kontinuierlichen Herstellung von ungesättigten Carbonsäureanhydriden wie aus dem Stand der Technik bekannt, siehe EP 2 032 519.
Figur 2 zeigt eine für das erfindungsgemäße Verfahren geeignete Apparatur.

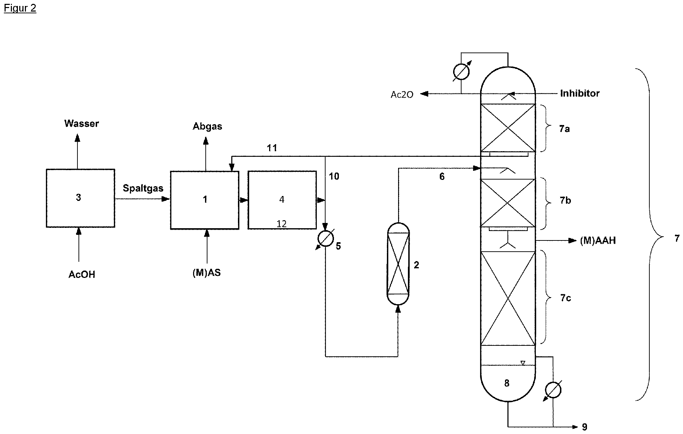

Das Keten wird in einem dem erfindungsmäßen Verfahren vorgeschalteten Schritt erzeugt, vgl. Reaktionsbereich (3) der Fig. 2. Das gewonnene Keten wird nach üblichen Methoden abgetrennt und mit einer ungesättigten Carbonsäure der allgemeinen Formel R-COOH (III), worin R die obengenannte Bedeutung hat, in einem ersten Reaktionsbereich oder Reaktor (1) umgesetzt (Verfahrensschritt (a) des erfindungsgemäßen Verfahrens). Dieser erste Reaktionsbereich (1) muss nicht zwingend mit den weiteren Bestandteilen der Apparatur verbunden sein, kann jedoch damit verbunden sein.

Die Umsetzung im ersten Reaktionsbereich wird bei Temperaturen im Bereich von 40 bis 100 °C, insbesondere bei 50 bis 90 °C, und besonders bevorzugt bei 70 bis 85 °C, durchgeführt.

Die erhaltene Roh-Anhydridmischung, bestehend aus symmetrischen und gemischten Anhydriden, wird in einem zweiten Reaktionsbereich oder Reaktor (2) weiter umgesetzt (Verfahrensschritt (b) des erfindungsgemäßen Verfahrens), wobei unter "umsetzen" die Einstellung des Gleichgewichtes zwischen allen Reaktanten zu verstehen ist. Der zweite Reaktionsbereich (2) kann außerhalb und/oder innerhalb der Rektifikationskolonne und/oder im ersten Reaktionsbereich (1) mit enthalten sein.

Vorzugsweise befindet sich der zweite Reaktionsbereich / Reaktionsbereich (2) außerhalb der Rektifikationskolonne.

Die Umsetzung im zweiten Reaktionsbereich wird bei Temperaturen im Bereich von 30 bis 120°C, insbesondere bei 40 bis 100 °C, und besonders bevorzugt bei 50 bis 80 °C, durchgeführt. Dabei ist die Reaktionstemperatur abhängig vom eingestellten Systemdruck. Falls Reaktionsbereich (2) innerhalb der Kolonne angeordnet ist, wird die Reaktion vorzugsweise im Druckbereich von 5 bis 100 mbar (absolut), insbesondere bei 10 bis 50 mbar (absolut) und besonders bevorzugt bei 20 bis 40 mbar (absolut) durchgeführt.

Falls sich Reaktionsbereich (2) außerhalb der Kolonne und separat von Reaktionsbereich (1) befindet, können dort andere Druck- und Temperaturverhältnisse gewählt werden. Das hat den Vorteil, dass die Reaktionsparameter des Reaktors (2) unabhängig von den Betriebsbedingungen in der Kolonne und im Reaktionsbereich (1) eingestellt werden können.

Die Reaktionsdauer hängt von der Reaktionstemperatur ab; die Verweilzeit im Reaktionsbereich (2) bei einmaligem Durchgang beträgt vorzugsweise 0,5 bis 15 Minuten und besonders bevorzugt 1 bis 5 Minuten.

Die Reaktionsmischung kann neben den Reaktanten weitere Bestandteile, wie beispielsweise Lösungsmittel, Katalysatoren und Polymerisationsinhibitoren umfassen.

Zwischen Verfahrensschritt (a) in einem ersten Reaktionsbereich (1) und Verfahrensschritt (b) in einem zweiten Reaktionsbereich (2) kann eine Zwischenlagerung der Roh-Anhydridmischung erfolgen. Bei einer solchen Zwischenlagerung kann sich das dynamische Gleichgewicht aller Reaktanten ausbilden.

Schließlich werden die symmetrischen Anhydride der allgemeinen Formeln (I) und (II) im Wege einer Rektifikation (vgl. Rektifikationskolonne (7) in Fig. 2) isoliert, vgl. Verfahrensschritt (c) des erfindungsgemäßen Verfahrens. Bei der Rektifikation anfallendes Mischanhydrid wird in Verfahrensschritt (d) in den ersten und/oder in den zweiten Reaktionsbereich zurückgeführt (vgl. Strom (11) in Fig. 2).

Im zweiten Reaktionsbereich (vgl. Reaktionsbereich (2) der Fig. 2) ist vorzugsweise mindestens ein Katalysator anwesend.

Falls ein Katalysator innerhalb des zweiten Reaktionsbereiches (2) eingesetzt wird und sich dieser zweite Reaktionsbereich innerhalb der Rektifikationskolonne befindet, kann der Katalysator grds. in jedem Bereich der Rektifikationskolonne eingesetzt werden, bevorzugt jedoch im mittleren Bereich.

Vorzugsweise wird der Katalysator jedoch in einem außerhalb der Kolonne befindlichen und separat von Reaktionsbereich (1) angeordneten Reaktionsbereich (2) bereitgestellt. Diese Anordnung des Katalysatorbereichs ist bevorzugt. Die Roh-Anhydridmischung wird vorzugsweise ständig durch den Katalysatorbereich geleitet. Hierdurch entsteht kontinuierlich das ungesättigte Carbonsäureanhydrid der Formel (I), beispielsweise (Meth)acrylsäureanhydrid, sowie ein Carbonsäureanhydrid der Formel (II), welches dem symmetrischen Anhydrid der Carbonsäure aus Formel (III) entspricht, beispielsweise Essigsäureanhydrid, das als Wertstoff veräußert werden kann.

Bevorzugt können homogene Katalysatoren dem Kopf der Kolonne oder dem Reaktionsbereich (2) zugegeben werden. Als homogene Katalysatoren besonders geeignet sind Magnesiumbromid (vorzugsweise wasserfrei) und Triflatsalze aller seltenen Erden.

Besonders bevorzugt werden in dem Reaktionsbereich (2) heterogene Katalysatoren verwendet. Als heterogene Katalysatoren besonders geeignet sind saure Festbettkatalysatoren, insbesondere saure Ionenaustauscher (Kationenaustauscher).

Zu den besonders geeigneten sauren Ionenaustauschern gehören insbesondere Kationenaustauscherharze wie sulfonsäuregruppenhaltige Styrol-Divinylbenzolpolymere. Geeignete Kationenaustauscherharze können kommerziell von Rohm&Haas unter der Handelsbezeichnung Amberlyst^{®}, von Dow unter der Handelsbezeichung Dowex^{®} und von Lanxess unter der Handelsbezeichnung Lewatit^{®} erhalten werden.

Die Katalysatormenge in L beträgt vorzugsweise 1/10 bis das 2fache, besonders bevorzugt 1/5 bis 1/2, der zu produzierenden Menge an ungesättigten Carbonsäureanhydrid der Formel I in L/h.

Zur Stabilisierung von Reaktanten, Zwischenprodukten und/oder Produkten können Stabilisatoren/Polymerisationsinhibitoren eingesetzt werden.

Zu den bevorzugt einsetzbaren Polymerisationsinhibitoren gehören unter anderem Octadecyl-3-(3,5-di-tert-butyl-4-hydroxyphenyl)-propionat, Phenothiazin, Hydrochinon, Hydrochinonmonomethylether, 4-Hydroxy-2,2,6,6-tetramethylpiperidinooxyl (TEMPOL), 2,4-Dimethyl-6-tert-butylphenol, 2,6-Di-tert-butylphenol, 2,6-Di-tert-butyl-4-methylphenol, para-substituierte Phenylendiamine wie z. B. N,N'-Diphenyl-p-phenylendiamin, 1,4-Benzochinon, 2,6-Di-tert-butyl-alpha-(dimethylamino)-p-cresol, 2,5-Di-tert-butylhydrochinon oder Gemische aus zwei oder mehreren dieser Stabilisatoren. Ganz besonders bevorzugt ist Phenothiazin.

Die Zudosierung des Inhibitors kann in den Feed der ungesättigten Carbonsäure der allgemeinen Formel (III), vor den Reaktionsbereich (1), vor den Reaktionsbereich (2) und/oder in die Rektifikationskolonne, vorzugsweise an deren Kopf, erfolgen.

Weiter kann im Rektifikationsschritt (c) eine hochsiedende, inerte Substanz mit einem Siedepunkt höher als die Siedepunkte der an der Reaktion beteiligten Komponenten als Siedeöl verwendet werden, um die destillative Abtrennung des gebildeten Säureanhydrids ohne Polymerisation zu gewährleisten.

Der Siedepunkt des Siedöls sollte aber auch nicht zu hoch sein, um die thermische Belastung des gebildeten Säureanhydrids zu reduzieren. Das Siedeöl befindet sich im Sumpf der Kolonne, um lange Verweilzeiten des polymerisationsanfälligen Zielproduktes zu vermeiden.

Im Allgemeinen liegt die Siedetemperatur des Siedeöls bei Normaldruck (1013 mbar) bei 200 bis 400 °C, insbesondere bei 240 bis 290 °C.

Geeignete Siedeöle sind u.a. höherkettige unverzweigte Paraffine mit 12 bis 20 Kohlenstoffatomen, aromatische Verbindungen wie Diphyl (eutektische Mischung aus 75 % Biphenyloxid und 25 % Biphenyl), alkylsubstituierte Phenole oder Naphthalinverbindungen, Sulfolan (Tetrahydro-thiophen-1,1-dioxid) oder Mischungen aus diesen.

Geeignete Beispiele sind die nachfolgend aufgeführten Siedeöle:

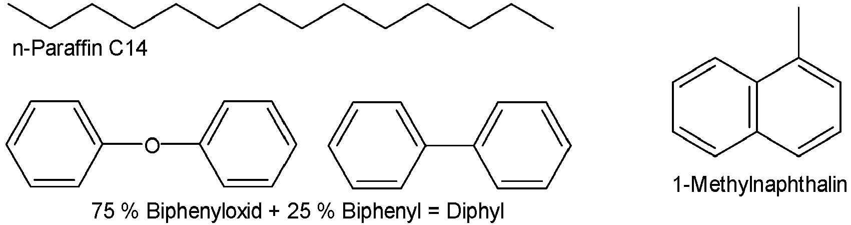

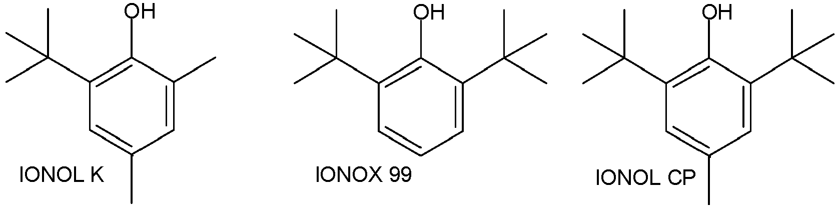

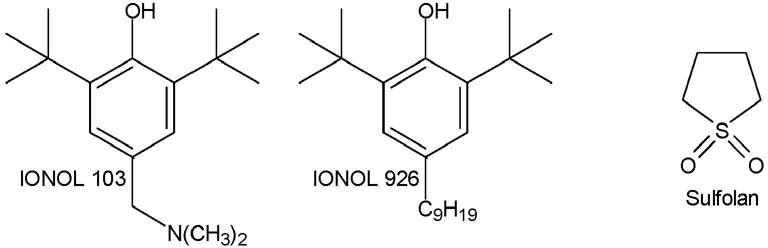

Besonders bevorzugt werden 2,6-di-tert-Butyl-para-cresol, 2,6-di-tert-Butyl-phenol, Sulfolan, Diphyl oder Mischungen aus diesen eingesetzt, ganz besonders bevorzugt Sulfolan.

Für die Aufreinigung der Rohanhydridmischung gemäß verfahrensschritt (c) der vorliegenden Erfindung wird eine Rektifikationskolonne verwendet. Vorzugsweise wird eine Rektifikationskolonne verwendet, die im oberen, mittleren und unteren Bereich je 5 bis 15 Trennstufen besitzt. Bevorzugt beträgt die Zahl der Trennstufen im oberen Bereich 10 bis 15 und im mittleren und unteren Bereich 8 bis 13.

Als Anzahl der Trennstufen wird in der vorliegenden Erfindung die Anzahl der Böden bei einer Bodenkolonne multipliziert mit dem Bodenwirkungsgrad oder die Anzahl der theoretischen Trennstufen im Fall einer Packungskolonne oder eine Kolonne mit Füllkörpern bezeichnet.

Beispiele für eine Rektifikationskolonne mit Böden beinhalten solche wie Glockenböden, Siebböden, Tunnelböden, Ventilböden, Schlitzböden, Sieb-Schlitzböden, Sieb-Glockenböden, Düsenböden, Zentrifugalböden, für eine Rektifikationskolonne mit Füllkörpern solche wie Raschig-Ringe, Lessing-Ringe, Pall-Ringe, Berl-Sättel, Intalox Sättel und für eine Rektifikationskolonne mit Packungen solche wie vom Typ Mellapak (Sulzer), Rombopak (Kühni), Montz-Pak (Montz) und Packungen mit Katalysatortaschen, beispielsweise Katapak (Sulzer).

Eine Rektifikationskolonne mit Kombinationen aus Bereichen von Böden, aus Bereichen von Füllkörpern und/oder aus Bereichen von Packungen kann ebenso verwendet werden.

Bevorzugt wird eine Rektifikationskolonne mit Füllkörpern und/oder Packungen eingesetzt.

Die Rektifikationskolonne kann aus jedem hierfür geeigneten Material hergestellt werden. Hierzu gehören u.a. Edelstahl sowie inerte Materialien.

Die Rückführung nicht umgesetzten Mischanhydrids in den Reaktionsbereich in Schritt (d) erfolgt zum Beispiel mittels einer Pumpe.

Aus dem Kolonnensumpf können Hochsieder wie zugesetzte Inhibitoren durch übliche Methoden ausgeschleust werden, beispielsweise durch einen Dünnschichtverdampfer oder einen für ähnliche Aufgaben vorgesehenen Apparat, der verdampfende Stoffe in die Rektifikationskolonne zurückführt und nicht verdampfende Hochsieder oder Salze ausschleust.

Das erfindungsgemäße Verfahren kann kontinuierlich oder semi-kontinuierlich betrieben werden.

Eine bevorzugte Ausführungsform des erfindungsgemäßen Verfahrens ist in Fig. 2 schematisch dargestellt.

Die Herstellung von Keten (hier: CH₂=C=O) erfolgt im Reaktionsbereich (3) nach den oben beschriebenen Verfahren.

Die nachfolgende Umsetzung dieses Ketens mit (Meth)acrylsäure (= (M)AS) erfolgt in einem ersten Reaktionsbereich (1). Für die Herstellung von (Meth)acrylsäureanhydrid aus Keten CH₂=C=O und (Meth)acrylsäure beträgt die Reaktionstemperatur im Reaktionsbereich (1) vorzugsweise 40 bis 100 °C, besonders bevorzugt 50 bis 90 °C und ganz besonders bevorzugt 70 bis 85 °C.

Die aus Verfahrensschritt (a) im Reaktionsbereich (1) erhaltene Roh-Anhydridmischung (4) wird nun dem zweiten Reaktionsbereich (2) zugeführt, wo die weitere Umsetzung gemäß verfahrensschritt 8b) erfolgt.

Die Temperatur der Reaktanten kann dabei über einen Wärmetauscher (5) in der Zuführung eingestellt werden. Der Reaktor (2) ist vorzugsweise ein Strömungsrohrreaktor, der einen Festbettkatalysator enthält. Als Festbettkatalysator wird bevorzugt ein saurer Ionenaustauscher eingesetzt.

Der Reaktorabstrom (6) aus dem zweiten Reaktionsbereich (2) wird in die Rektifikationskolonne (7) eingespeist, vorzugsweise unterhalb des Rücklaufstromes aus dem oberen Bereich (7a) der Kolonne. In der Kolonne (7) findet die Trennung der Komponenten statt.

Zur Vermeidung von Polymerisation wird vorzugsweise sowohl am Kopf der Kolonne (7) und in den (M)AS-Feed mindestens ein Polymerisationsinhibitor zudosiert.

Im oberen Bereich (7a) wird das leichtsiedende Essigsäureanhydrid von dem Mittelsieder (gemischtes Anhydrid) abgetrennt, am Kopf abgezogen und als Wertstoff gewonnen.

Im mittleren Bereich (7b) der Kolonne findet die Trennung Mittelsieder gegen (Meth)acrylsäureanhydrid (= (M)AAH) statt, wobei zwischen dem mittleren und unteren Teil (M)AAH bevorzugt gasförmig abgezogen wird. Im unteren Bereich (7c) der Kolonne wird (M)AAH von dem im Sumpf befindlichen Siedeöl (8) getrennt. Im Sumpf befindliche Hochsieder können durch übliche Methoden ausgeschleust werden (9), beispielsweise durch einen Dünnschichtverdampfer oder einen ähnliche Aufgaben verrichtenden Apparat, der verdampfende Stoffe in die Rektifikationskolonne zurückführt und nicht verdampfende Hochsieder ausschleust.

Der aus dem oberen Bereich (7a) resultierende Flüssigkeitsstrom wird vollständig aus der Kolonne abgezogen und getrennt gesammelt. Das Mischanhydrid (4) wird als Kreislaufstrom (10) dem Reaktor (2) zugeführt. Alternativ kann der Kreislaufstrom (10) vollständig oder teilweise auch dem Reaktionsbereich (1) über Leitung (11) zugeführt werden, sofern beide Reaktionsschritte in einer gemeinsamen Anlage durchgeführt werden. Alternativ kann das Mischanhydrid (4) in einem Tank zwischengelagert oder transportiert werden, sofern die Anlagenteile räumlich voneinander getrennt sind.

Die nachfolgenden Beispiele veranschaulichen das erfindungsgemäße Verfahren ohne dieses auf diese zu beschränken.

### Beispiele

### Vergleichsbeispiel 1:

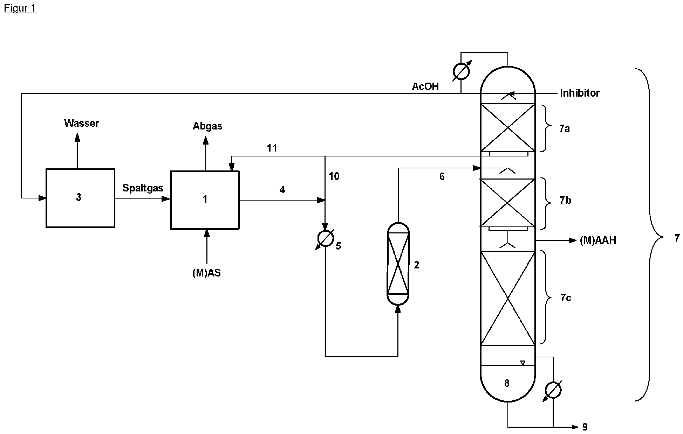

Eine Anlage zur Herstellung von (Meth)acrylsaueranhydrid wie in EP 2 032 519 beschrieben, wurde in einen stationären und stabilen Zustand der maximalen Auslastung gebracht und hier die Parameter festgehalten bei der ein Betrieb gerade noch möglich ist. Die Anlage ist in Fig. 1 dargestellt.

Hierbei wurden in den Reaktor 2 im stationären Betrieb ein Feed von 363.2g/h MAS und 255.2g/h Essigsäureanhydrid eingetragen. Über den Kolonnenabzug konnten hierbei 309.7g/h MAAH mit einer Reinheit grösser 99% entnommen werden. Am Kopf der Kolonne fiel ein Destillat mit 237.5g/h an welches in seiner Zusammensetzung 91,6% Essigsäure, 6,3% Essigsäureanhydrid und 2,1% Methacrylsäure enthielt. Der Kondensator am Kopf der Kolonne kann bei maximaler Auslastung das Destillat auf 18C abkühlen, der Verlust über das Abgas hinter dem Kondensator betrug 32g/h. Zwischen den Packungselementen (7a, b und c) befindliche Thermoelemente zeichnen mit den Kopf- und Sumpftemperaturen sowie der Produktaustragstemperatur ein kontinuierliches Temperaturprofil der Kolonne auf.

### Beispiel 1:

Die Anlage aus Vergleichsbeispiel 1 wurde umgebaut wie in Figur 2 gezeigt. Als Feed für die Kolonne wird säurefreies Mischanhydrid (aus Keten und Methacrylsäure) verwendet. Der Feed wird so lange erhöht bis vergleichbare Belastungsparameter in der Kolonne erreicht sind wie im Vergleichsbeispiel 1. Anschließend wir die Kolonne mit diesen Fahrparametern stationär betrieben. Im stationären Betrieb werden 1000g/h Mischanhydrid über Leitung 6 in die Kolonne eingetragen. Hieraus können 598g/h Methacrylsäureanhydrid identischer Qualität wie im Vergleichsbeispiel 1 isoliert werden. Am Kopf der Kolonne fallen 370.6g/h Destillat von Essigsäureanhydrid mit einer Reinheit von >99% an. Der Kondensator am Kopf der Kolonne kann bei maximaler Auslastung das Destillat auf 22°C abkühlen, der Verlust über das Abgas hinter dem Kondensator betrug 29.8g/h (Das Abgas besteht aus dem reinen Anhydrid der Essigsäure). Eine Steigerung der Eintragsmenge hat wie im Vergleichsbeispiel 1 einen starken Anstieg der Abgasverluste zur Folge, so dass ein vergleichbarer Betriebspunkt gefunden wurde.

Die berechnete Kolonnenbelastung und Berieselungsdichte ist mit den stationären Betriebsparametern in Tabelle 1 für den direkten Vergleich zu entnehmen und belegt einen Betrieb der Kolonne unter vergleichbaren Bedingungen. Die Kapazität der Anlage konnte um den Faktor 1.93 gesteigert und gleichzeitig der Wertstoff Essigsäureanhydrid gewonnen werden.

**Tabelle 1: Vergleich der Betriebsparameter von Vergleichsbeispiel und erfindungsgemäßem Beispiel**

| | Vergleichsbeispiel 1 | | | Beispiel 1 | | |
|---|---|---|---|---|---|---|
| Position | Temp. Kolonne [C] | F-Faktor | Berieselungsdichte | Temp. Kolonne [C] | F-Faktor | Berieselungsdichte |
| Condenser | 18.00 | 1.39 | 1.39 | 22.00 | 1.41 | 1.61 |
| 7a | 59.17 | 1.11 | 1.13 | 46.31 | 1.39 | 1.47 |
| reactor in | 78.31 | 0.99 | 1.42 | 78.75 | 1.43 | 2.05 |
| 7b | 92.57 | 1.07 | 1.57 | 87.46 | 1.41 | 2.11 |
| 7c | 103.41 | 1.24 | 1.80 | 103.44 | 1.58 | 2.27 |
| Reboiler | 106.27 | 1.14 | 1.76 | 130.47 | 1.20 | 1.50 |
| | | | | | | |
| MAAH produziert | 309.7 | [g/h] | | 599.0 | [g/h] | |

| **Feedströme** | | | | | | |
|---|---|---|---|---|---|---|
| MAS | 363.2 | [g/h] | | | [g/h] | |
| Ac2O | 255.2 | [g/h] | | | [g/h] | |
| AcOMA | 0.0 | [g/h] | | 1000.0 | [g/h] | |

| **Kopfprodukt** | | | | | | |
|---|---|---|---|---|---|---|
| Destillat | 237.5 | [g/h] | | 370.6 | [g/h] | |
| AcOH | 91.6% | [wt%] | | 0.0% | [wt%] | |
| Ac2O | 6.3% | [wt%] | | 100.0% | [wt%] | |
| MAS | 2.1% | [wt%] | | 0.0% | [wt%] | |
| Abgasverlust | 32.0 | [g/h] | | 29.8 | [g/h] | (100 % Ac2O) |

## Patentansprüche

**1.** Verfahren zur gleichzeitigen Herstellung von symmetrischen Carbonsäureanhydriden der allgemeinen Formeln
R-C(O)-O-C(O)-R (I)
und
R'R"HC-C(O)-O-C(O)-CHR'R" (II)
in der R einen ungesättigten organischen Rest mit 2 bis 12 C-Atomen darstellt,
worin R' und R" gleich oder verschieden sind und Wasserstoff oder einen C₁ bis C₄-Alkylrest darstellen,
und worin R' und R" verschieden von R sind
durch Reaktion einer ungesättigten Carbonsäure der allgemeinen Formel
R-COOH (III),
in der R die oben angegebene Bedeutung hat,
mit einem Keten der allgemeinen Formel
R'R"C=C=O (IV),
in der R' und R" die oben angegebenen Bedeutungen haben,
**dadurch gekennzeichnet, dass**
(a) die ungesättigte Carbonsäure (III) in einem ersten Reaktionsbereich mit dem Keten (IV) zu einer Roh-Anhydridmischung umgesetzt wird,
(b) die in Schritt (a) erhaltene Roh-Anhydridmischung in einem zweiten Reaktionsbereich, vorzugsweise in Gegenwart mindestens eines Katalysators, weiter umgesetzt wird,
(c) die symmetrischen Anhydride (I) und (II) im Wege einer Rektifikation aus der in Schritt (b) erhaltenen Mischung isoliert werden, und
(d) bei der Rektifikation in Schritt (c) anfallendes, nicht umgesetztes Mischanhydrid in den ersten Reaktionsbereich und/oder in den zweiten Reaktionsbereich zurückgeführt wird.

**2.** Verfahren gemäß Anspruch 1, **dadurch gekennzeichnet, dass** das molare Verhältnis von ungesättigter Carbonsäure der Formel (III) zu Keten der Formel (IV) 1:4 bis 1:0,5, vorzugsweise 1:1, beträgt.

**3.** Verfahren gemäß einem der Ansprüche 1 oder 2, **dadurch gekennzeichnet, dass** das ungesättigte Carbonsäureanhydrid der Formel (I) (Meth)acrylsäureanhydrid ist, hergestellt durch Reaktion von einem Keten der Formel CH₂=C=O und (Meth)acrylsäure.

**4.** Verfahren gemäß Anspruch 3, **dadurch gekennzeichnet, dass** das Carbonsäureanhydrid der Formel (II) Essigsäureanhydrid ist.

**5.** Verfahren gemäß Anspruch 4, **dadurch gekennzeichnet, dass** das (Meth)acrylsäureanhydrid in der für Verfahrensschritt (c) verwendeten Rektifikationskolonne zwischen dem mittleren und dem unteren Bereich der Rektifikationskolonne erhalten wird und das Essigsäureanhydrid am Kolonnenkopf abgezogen wird.

**6.** Verfahren gemäß einem der vorhergehenden Ansprüche, **dadurch gekennzeichnet, dass** der zweite Reaktionsbereich räumlich getrennt von ersten Reaktionsbereich ist und außerhalb der in der für Verfahrensschritt (c) verwendeten Rektifikationskolonne angeordnet ist.

**7.** Verfahren gemäß einem der vorhergehenden Ansprüche, **dadurch gekennzeichnet, dass** die Umsetzung im zweiten Reaktionsbereich bei Temperaturen im Bereich von 30 bis 120 °C, insbesondere bei 40 bis 100 °C, und besonders bevorzugt bei 50 bis 80 °C, durchgeführt wird.

**8.** Verfahren gemäß einem der vorhergehenden Ansprüche, **dadurch gekennzeichnet, dass** zwischen Verfahrensschritt (a) und Verfahrensschritt (b) eine Zwischenlagerung der Roh-Anhydridmischung erfolgt.

**8.** Verfahren gemäß einem der vorhergehenden Ansprüche, **dadurch gekennzeichnet, dass** in dem zweiten Reaktionsbereich ein heterogener Katalysator verwendet wird.

**10.** Verfahren gemäß Anspruch 8, **dadurch gekennzeichnet, dass** in dem zweiten Reaktionsbereich ein saurer Festbettkatalysator verwendet wird.

**11.** Verfahren gemäß einem der Ansprüche 8 oder 9, **dadurch gekennzeichnet, dass** in dem zweiten Reaktionsbereich ein Kationenaustauscher als Katalysator verwendet wird.

**12.** Verfahren gemäß einem der vorhergehenden Ansprüche, **dadurch gekennzeichnet, dass** im Rektifikationsschritt (c) eine hochsiedende, inerte Substanz mit einem Siedepunkt höher als die Siedepunkte der an der Reaktion beteiligten Komponenten als Siedeöl verwendet wird.

**13.** Verfahren gemäß Anspruch 12, **dadurch gekennzeichnet, dass** als Siedeöl 2,6-di-tert-Butyl-para-cresol, 2,6-di-tert-Butyl-phenol, Sulfolan oder Diphyl oder Mischungen aus diesen verwendet werden.

**14.** Verfahren gemäß einem der Ansprüche 12 oder 13, **dadurch gekennzeichnet, dass** als Siedeöl Sulfolan verwendet wird.

**15.** Verfahren gemäß einem der vorhergehenden Ansprüche, **dadurch gekennzeichnet, dass** aus dem Kolonnensumpf in der für Verfahrensschritt (c) verwendeten Rektifikationskolonne hochsiedende Komponenten ausgeschleust werden und verdampfende Stoffe in die Kolonne zurückgeführt werden.
